# EUROPEAN PATENT APPLICATION

(11) **EP 3 499 485 A1**
(43) Date of publication of application: **19.06.2019**
(21) Application number: 18213203.5
(22) Date of filing: 17.12.2018
(51) Int. Cl.: G09B 23/28

(54) **SYSTEMS AND METHODS FOR ASSESSING AIR LEAKS IN LUNG TISSUE**

(30) Priority: 18.12.2017 US 201762599910 P; 03.12.2018 US 201816208205
(71) Applicant: Covidien LP, Mansfield, MA 02048 (US)
(72) Inventor: FRUSHOUR, Scott E.M., Boulder, Colorado 80304 (US); HODGKINSON, Gerald N., Guilford, Connecticut 06437 (US)
(74) Representative: Maschio, Antonio

(57) **Abstract**

A medical system for testing air leaks in tissue includes a housing unit, a tubular member, a source of negative pressure, a source of positive pressure, and a pressure gauge. The housing unit defines an interior chamber configured for receipt of at least a portion of an organ. The tubular member is coupled to the housing unit and includes a first end portion configured to be coupled to the portion of the organ. The source of negative pressure is configured to be in fluid communication with the interior chamber of the housing unit. The source of positive pressure is configured to be in fluid communication with the portion of the organ via attachment to a second end portion of the tubular member. The pressure gauge is in communication with the interior chamber of the housing unit and the tubular member to determine a pressure differential therebetween.

## Description

### BACKGROUND

### Technical Field

The present disclosure relates to surgical systems, and more particularly, to systems and methods for assessing air leaks in lung tissue.

### Description of Related Art

To treat certain diseases of the lung, the diseased or malfunctioning lung tissue may be removed or resected. After resecting the subject lung tissue, a surgical instrument, such as a surgical stapler, an electrosurgical forceps, or the like, may be utilized to ligate the lung tissue and effectuate a seal. Occasionally, despite having been properly performed, a complete seal may not be created, resulting in air leaks.

During a simulated surgical procedure, the extent of an air leak in the subject lung tissue may be used to test the effectiveness of the simulated surgical procedure and the particular surgical instrument used. Accordingly, it would be beneficial to provide an improved system and method for assessing air leaks in simulated lung tissue.

### SUMMARY

In one aspect of the present disclosure, a medical system for testing air leaks in tissue is provided. The medical system includes a housing unit, a tubular member, a source of negative pressure, a source of positive pressure, and a first pressure gauge. The housing unit defines an interior chamber configured for receipt of at least a portion of an organ. The tubular member is coupled to the housing unit and includes a first end portion configured to be coupled to the portion of the organ. The source of negative pressure is configured to be in fluid communication with the interior chamber of the housing unit. The source of positive pressure is configured to be in fluid communication with the portion of the organ via attachment to a second end portion of the tubular member. The first pressure gauge is in communication with the interior chamber of the housing unit and the tubular member to determine a pressure differential therebetween.

In embodiments, the housing unit may be configured to simulate a chest cavity.

In embodiments, the housing unit may define a hole configured for receipt of a trocar.

In embodiments, the housing unit may be configured to transition between a contracted state, in which the interior chamber of the housing unit has a first volume, and an expanded state, in which the interior chamber of the housing unit has a second volume, greater than the first volume.

In embodiments, the medical system may further include a simulated lung disposed within the interior chamber of the housing unit. The first end portion of the tubular member may be fluid communication with the simulated lung. An exterior of the simulated lung may be substantially sealed from the atmosphere by the housing unit. The medical system may further include a respiratory controller configured to control inspiration and expiration of air into and out of a plurality of airways of the simulated lung.

In embodiments, the medical system may further include a second and third pressure gauge. The first pressure gauge may be in fluid communication with the tubular member for determining a pressure within the tubular member. The third pressure gauge may be in fluid communication with the interior chamber of the housing unit for determining a pressure within the interior chamber of the housing unit.

In another aspect of the present disclosure, a method of testing air leaks in lung tissue is provided. The method includes removing a fluid from an interior chamber of a housing unit; delivering a fluid to lung tissue disposed within the housing unit via a tubular member that is coupled to the housing unit and in fluid communication with the lung tissue; and determining a pressure differential between the interior chamber of the housing unit and the lung tissue using a first pressure gauge in communication with the interior chamber of the housing unit and the lung tissue.

Methods may further include inserting a trocar through a hole defined through the housing unit, and performing a surgical procedure on the at least one lung through the trocar.

In methods, the pressure differential may be determined after the surgical procedure is performed.

In methods, the surgical procedure may include contracting the lung tissue, stapling and/or sealing a piece of the lung tissue, and expanding the lung tissue.

Methods may further include transitioning the housing unit between a contracted state, in which the interior chamber of the housing unit has a first volume, and an expanded state, in which the interior chamber of the housing unit has a second volume, greater than the first volume.

Methods may further include determining a pressure within the tubular member via a second pressure gauge, and determining a pressure within the interior chamber of the housing unit via a third pressure gauge.

Methods may further include comparing the determined pressure differential to a known pressure differential associated with healthy lung tissue.

Further details and aspects of exemplary embodiments and methods of the present disclosure are described in more detail below with reference to the appended figures.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

Various aspects and features of the present disclosure are described hereinbelow with references to the drawings, wherein:
FIG. 1 is a perspective view of a medical system for assessing air leaks in lung tissue in accordance with the principles of the present disclosure; and
FIG. 2 is a schematic illustration of the medical system of FIG. 1.

### DETAILED DESCRIPTION

The present disclosure is directed to systems and methods for enhanced approaches to assessing the extent of air leaks in simulated lung tissue after performing a simulated surgical procedure on the subject lung tissue.

Embodiments of the present disclosure are now described in detail with reference to the drawings in which like reference numerals designate identical or corresponding elements in each of the several views. As used herein, the term "clinician" refers to a doctor, a nurse, support personnel or any other person that may use the medical system described herein to train on surgical techniques or test the effectiveness of surgical instruments. Throughout this description, the term "proximal" will refer to the portion of the device or component thereof that is closer to the clinician and the term "distal" will refer to the portion of the device or component thereof that is farther from the clinician. Additionally, in the drawings and in the description that follows, terms such as front, rear, upper, lower, top, bottom, and similar directional terms are used simply for convenience of description and are not intended to limit the disclosure. In the following description, well-known functions or constructions are not described in detail to avoid obscuring the present disclosure in unnecessary detail.

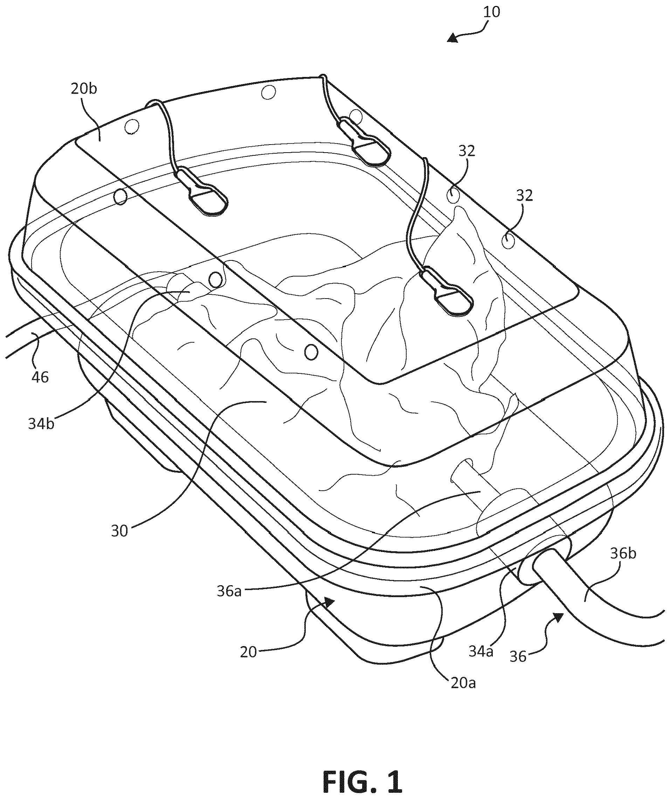

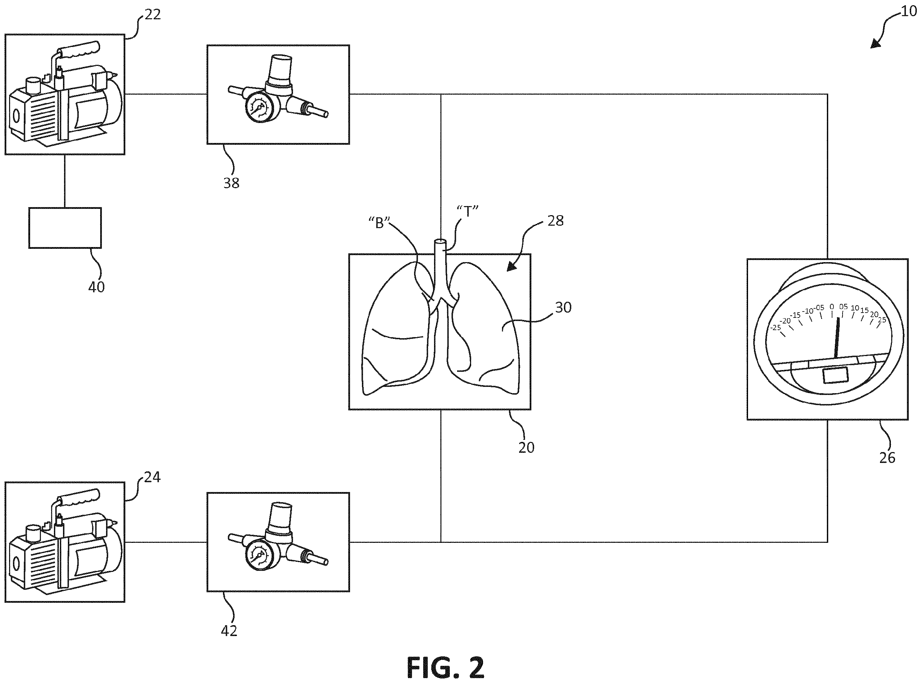

With reference to FIGS. 1 and 2, a medical system for determining air leaks in tissue, such as, for example, lung tissue, is provided and generally designated 10. The medical system 10 may be used in training clinicians for bronchoscopy, percutaneous procedures, or other thoracic procedures, comparing the effectiveness of particular surgical procedures in stapling, sealing, or suturing resected lung tissue, or comparing the effectiveness of a surgical instrument in stapling, sealing, or suturing resected lung tissue. The medical system 10 generally includes a housing unit 20, a source of positive pressure 22, a source of negative pressure 24, and a pressure sensor 26 for determining a pressure differential between animal lung 30 disposed within the housing unit 20 and an interior chamber 28 defined within the housing unit 20, as will be described herein.

The housing unit 20 has a generally rectangular configuration and is configured to house a set of animal lungs 30. In embodiments, the housing unit 20 may be designed to visually resemble a human body. In embodiments, the housing unit 20 may be configured to house any suitable organ, such as, for example, a heart, and may assume any suitable shape. The housing unit 20 may simulate the function of a chest cavity by being transitionable between contracted and expanded states, and may be equipped with rib-like structures (not shown) to enhance the lifelike appearance of the housing unit 20. The housing unit 20 may be fabricated from a flexible material, such as, for example, rubber, that expands upon exceeding a threshold internal pressure, and contracts upon dropping below the threshold internal pressure. In some embodiments, a single lung may be housed within housing unit 20. The housing unit 20 may have an opaque outer-surface or a clear outer-surface or an outer surface having a changeable opacity to provide a clear option for when clinicians are first familiarizing themselves with the system 10, and an opaque option as familiarity is developed and a more life like setting with limited visibility of actions is desired.

The housing unit 20 defines a plurality of ports or holes 32 therethrough that are dimensioned for receipt of trocars, surgical instruments, or the like. The holes 32 provide substantially air tight access into the interior chamber 28 of the housing unit 30 for conducting simulated laparoscopic surgical procedures on the housed lung tissue 30. The housing unit 20 has a first end portion 20a defining a first opening 34a, and a second end portion 20b defining an opening 34b. The first opening 34a provides access for the source of positive pressure 22 to the lung tissue 30, and the second opening 34b provides access for the source of negative pressure 24 to the interior chamber 28 of the housing unit 20.

Tissue of an organ, such as, for example, lung tissue 30 may be housed within the housing unit 20. It is contemplated that a whole organ or portions of an organ may be disposed within the housing unit 20. In embodiments, the lung tissue 30 may be any type of lung tissue that is similar anatomically to human lungs, such as ovine or porcine lungs. The lung tissue 30 defines a plurality of airways in fluid communication with the positive pressure source 22 while being housed within the housing unit 20. The exterior of the lungs 30 are substantially sealed from the atmosphere by the housing unit 20 forming an analogous arrangement to human and animal lungs sealed within the chest cavity but internally open to a source of air.

The medical system 10 includes a tubular member or hose 36 interconnecting the lung tissue 30 and the source of positive pressure 22. The tubular member 36 extends through the opening 34a of the housing unit 24 and is coupled to the first end portion 20a of the housing unit 20. The tubular member 36 has a first end portion 36a dimensioned for receipt in a trachea "T" or bronchus "B" of the lung tissue 30, and a second end portion 36b for receiving a fluid, such as, for example, air, from the source of positive pressure 22. In embodiments, instead of using the tubular member 36, the opening 24a defined in the first end portion 20a of the housing unit 20 may be configured for the sealed passage of the trachea "T" or bronchus "B" of the lung tissue 30. Upon interconnecting the first end portion 36a of the tubular member 36 with the trachea "T" or bronchus "B" of the lung tissue 30, the tubular member 36 allows for the egress and ingress of fluid (e.g., air) between the airways in the lung tissue 30 and the source of positive pressure 22.

The second end portion 36b of the tubular member 36 is coupled to the source of positive air pressure 22, which may be an air pump device or a ventilator. The medical system 10 includes a pressure gauge 38 in communication with the air pump device 22 and the tubular member 36 for determining and controlling the amount of positive pressure output by the air pump device 22. In embodiments, the pressure gauge 38 may determine the air pressure within the tubular member 36 or the bronchus "B" or trachea "T" of the lung tissue 30.

The medical system 10 may include a respiratory controller 40 in communication with the air pump device 22. The respiratory controller 40 may be configured to control inspiration and expiration of air into and out of the plurality of airways of the lung tissue 30. The amount of air output by the air pump device 22 may be based on the respiratory controller 40. The lung tissue 30 may be filled with air via the positive pressure ventilation, such as by using the air pump device 22.

The source of negative pressure 24 is in fluid communication with the interior chamber 28 of the housing unit 20 via another tubular member or hose 46. The hose 46 extends through the opening 34b in the second end portion 20b of the housing unit 20. The source of negative pressure 24 may be a vacuum or a negative pressure air pump device. The negative pressure air pump device 24 withdraws air from within the interior chamber 28 of the housing unit 20 to simulate the internal environment of a thoracic cavity. When lung tissue 30 is disposed within the housing unit 20, the negative pressure air pump device 24 withdraws air from the internal chamber 28 of the housing unit 20, thereby permitting expansion of the lung tissue 30. The negative pressure air pump device 24 is in fluid communication with a pressure gauge 42 for determining and controlling the amount of negative pressure output by the negative air pump device 24. In embodiments, the pressure gauge 42 may also determine the air pressure within the interior chamber 28 of the housing unit 20.

The pressure differential gauge 26 of the medical system 10 is in communication with the interior chamber 28 of the housing unit 20 and the tubular member 36 to determine a pressure differential therebetween. In embodiments, the pressure differential gauge 26 may be coupled to both the second end portion 36b of the tubular member 36 and one of the ports 32 defined in the housing unit 20. The output of the pressure differential gauge 26 is representative of the existence and degree of any air leaks in the lung tissue 30. For example, if each of the positive and negative pressure air pump devices 22, 24 generate a respective pressure of + 1 inches of water ("inch wc") and - 1 inch wc, and the pressure differential gauge 26 indicates a pressure of 2 inch wc, then no air leaks are present in the lung tissue 30. If each of the positive and negative pressure air pump devices 22, 24 are generating, respectively, + 1 inch wc and - 1 inch wc of pressure, and the pressure differential gauge 26 indicates a pressure of 1.5 inch wc, an air leak is present in the lung tissue 30.

The medical system 10 may include a fluid controller (not shown) configured to control administration of bodily fluids to the lung tissue 30, which includes the vasculature of the lungs. The fluid controller may be connected to one or more fluid sources (not shown) and inject fluid, via a tube, into a certain location within the lung tissue 30 housed in the housing unit 20. The fluid controller may receive a fluid including, but not limited to, blood, mucus, or any other suitable fluid that may be relevant for the particular procedure being simulated for a clinician. Additionally, the fluid may include substitutes for blood and mucus, such as a red color fluid or tapioca pudding, respectively. The medical system 10 may be configured to increase blood/fluid flow rate and pressure. In embodiments, the medical system 10 may generate a pulsatile fluid flow of the fluids into the vasculature of the lung tissue 30 to simulate blood flow generated by a beating heart.

In embodiments, the medical system 10 may be used to detect fluid leaks along a staple line. For example, the lung tissue 30 may be resected and sealed using a surgical stapler, after which the fluid controller may be activated to release dyes (e.g., fluorescent or brightly-colored) into the lung tissue 30. In further embodiments, a coagulation cascade activator, such as, for example, calcium chloride, may be placed in the fluid surrounding the lung tissue 30 to simulate the coagulation cascade in the event that blood leaks from the lung tissue 30.

The medical system 10 may provide quantitative data about any air leaks present in lung tissue subjected to simulated surgical procedures. The quantitative data may be used in measuring the effectiveness of a clinician, comparing the effectiveness of particular surgical procedures in sealing resected lung tissue, or comparing the effectiveness of surgical instruments in sealing resected lung tissue. In use, lung tissue 30 is positioned within the interior chamber 28 of the housing unit 20 and the tubular member 36 is coupled to the bronchus "B" of the lung tissue 30. The lung tissue 30 may be intubated and selectively evacuated or contracted (e.g., air is removed) by activating a vacuum function of the positive air pump 22, or increasing the pressure within the interior chamber 28 of the housing unit 20. In some methods, the housing unit 20 may be contracted to decrease the volume of the interior chamber 28 therein, whereby the lung tissue contracts in turn.

With the lung tissue 30 in a contracted or compressed state, a surgical instrument (e.g., a surgical stapling instrument, a surgical suturing instrument, or an electrosurgical sealing instrument) is inserted through one of the holes 32 in the housing unit 20 and a piece of the lung tissue 30 may be resected and sealed using the surgical instrument. Upon sealing the lung tissue 30, the lung tissue 30 is expanded to an inflated state by activating the positive air pump device 22 and/or by activating the negative air pump device 24. In some methods, the housing unit 20 may be expanded to increase the volume of the interior chamber 28, whereby the lung tissue 30 expands in turn.

Pulmonary air leaks in the lung tissue 30 that are present after the simulated surgical procedure may be quantified using the pressure differential gauge 26 of the medical system 10. In particular, both the positive and negative air pump devices 22, 24 are activated to generate a threshold positive air pressure and a threshold negative air pressure, respectively. The threshold positive and negative air pressures generated by the respective pump devices 22, 24 may be equal and opposite. The positive air pump device 22 moves fluid (e.g., air) into the lung tissue 30 via the tubular member 36, and the negative air pump device 24 moves fluid (e.g., air) out of the interior chamber 28 of the housing unit 20 via the tube 46. The pressure differential gauge 26 determines the pressure differential between the interior chamber 28 of the housing unit 20 and the lung tissue 30.

The determined pressure differential is representative of the degree of air leaks in the lung tissue 30. A low determined pressure differential indicates that the surgical procedure was unsuccessful, whereas a high determined pressure differential indicates that the air leaks are nominal to non-existent. The determined pressure differential may be compared to known pressure differentials associated with lung tissue of different degrees of air leakage. For example, if the determined pressure differential corresponds to the pressure differential expected in lung tissue with severe air leakage, then the lung tissue 30 is presumed to likewise have severe air leakage. Similarly, if the determined pressure differential corresponds to the pressure differential expected in healthy lung tissue with nominal to non-existent air leakage, then the lung tissue 30 is presumed to likewise have nominal to non-existent air leakage.

While the preceding embodiments are described in terms of surgical procedures on animal lungs, those skilled in the art will realize that the same or similar devices, systems, and methods may be used in other lumen networks, such as, for example, the vascular, lymphatic, and/or gastrointestinal networks as well.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to similar or identical elements throughout the description of the figures. Further, as used herein and as will be understood by those skilled in the art, the term "fluid" may refer to liquid and/or gaseous substances.

The systems described herein may also utilize one or more controllers to receive various information and transform the received information to generate an output. The controller may include any type of computing device, computational circuit, or any type of processor or processing circuit capable of executing a series of instructions that are stored in a memory. The controller may include multiple processors and/or multicore central processing units (CPUs) and may include any type of processor, such as a microprocessor, digital signal processor, microcontroller, programmable logic device (PLD), field programmable gate array (FPGA), or the like. The controller may also include a memory to store data and/or instructions that, when executed by the one or more processors, causes the one or more processors to perform one or more methods and/or algorithms.

The invention may be described by reference to the following numbered paragraphs:-
1. A medical system for testing air leaks in tissue, comprising:
   a housing unit defining an interior chamber configured for receipt of at least a portion of an organ;
   a tubular member coupled to the housing unit and including a first end portion configured to be coupled to the portion of the organ, and a second end portion;
   a source of negative pressure configured to be in fluid communication with the interior chamber of the housing unit;
   a source of positive pressure configured to be in fluid communication with the portion of the organ via attachment to the second end portion of the tubular member; and
   a first pressure gauge in communication with the interior chamber of the housing unit and the tubular member to determine a pressure differential therebetween.
2. The medical system according to paragraph 1, wherein the housing unit is configured to simulate a chest cavity.
3. The medical system according to paragraph 1, wherein the housing unit defines at least one hole configured for receipt of a trocar.
4. The medical system according to paragraph 1, wherein the housing unit is configured to transition between a contracted state, in which the interior chamber of the housing unit has a first volume, and an expanded state, in which the interior chamber of the housing unit has a second volume, greater than the first volume.
5. The medical system according to paragraph 1, further comprising at least one simulated lung disposed within the interior chamber of the housing unit, the first end portion of the tubular member in fluid communication with the at least one simulated lung.
6. The medical system according to paragraph 5, wherein an exterior of the at least one simulated lung is substantially sealed from the atmosphere by the housing unit.
7. The medical system according to paragraph 5, further comprising a respiratory controller configured to control inspiration and expiration of air into and out of a plurality of airways of the at least one simulated lung.
8. The medical system according to paragraph 1, further comprising:
   a second pressure gauge in fluid communication with the tubular member for determining a pressure within the tubular member; and
   a third pressure gauge in fluid communication with the interior chamber of the housing unit for determining a pressure within the interior chamber of the housing unit.

## Claims

1. A medical system for testing air leaks in tissue, comprising:
a housing unit defining an interior chamber configured for receipt of at least a portion of an organ;
a tubular member coupled to the housing unit and including a first end portion configured to be coupled to the portion of the organ, and a second end portion;
a source of negative pressure configured to be in fluid communication with the interior chamber of the housing unit;
a source of positive pressure configured to be in fluid communication with the portion of the organ via attachment to the second end portion of the tubular member; and
a first pressure gauge in communication with the interior chamber of the housing unit and the tubular member to determine a pressure differential therebetween.

2. The medical system according to claim 1, wherein the housing unit is configured to simulate a chest cavity.

3. The medical system according to claim 1 or claim 2, wherein the housing unit defines at least one hole configured for receipt of a trocar.

4. The medical system according to any preceding claim, wherein the housing unit is configured to transition between a contracted state, in which the interior chamber of the housing unit has a first volume, and an expanded state, in which the interior chamber of the housing unit has a second volume, greater than the first volume.

5. The medical system according to any preceding claim, further comprising at least one simulated lung disposed within the interior chamber of the housing unit, the first end portion of the tubular member in fluid communication with the at least one simulated lung.

6. The medical system according to claim 5, wherein an exterior of the at least one simulated lung is substantially sealed from the atmosphere by the housing unit.

7. The medical system according to claim 5 or claim 6, further comprising a respiratory controller configured to control inspiration and expiration of air into and out of a plurality of airways of the at least one simulated lung.

8. The medical system according to any preceding claim, further comprising:
a second pressure gauge in fluid communication with the tubular member for determining a pressure within the tubular member; and
a third pressure gauge in fluid communication with the interior chamber of the housing unit for determining a pressure within the interior chamber of the housing unit.
